(19) Europäisches Patentamt
European Patent Office
Office européen des brevets

(11) Veröffentlichungsnummer: **0 217 089 B1**

# (12) EUROPÄISCHE PATENTSCHRIFT

(45) Veröffentlichungstag der Patentschrift: **19.04.89**

(21) Anmeldenummer: **86111311.6**

(22) Anmeldetag: **15.08.86**

(51) Int. Cl.⁴: **C07C 41/28**, C07C 43/14, B01J 29/18, B01J 29/28, B01J 29/06

## (54) Verfahren zur Herstellung ungesättigter Ether durch katalytische Dealkoxylierung von geminalen Di-alkoxy-verbindungen.

(30) Priorität: **02.10.85 DE 3535128**

(43) Veröffentlichungstag der Anmeldung: **08.04.87 Patentblatt 87/15**

(45) Bekanntmachung des Hinweises auf die Patenterteilung: **19.04.89 Patentblatt 89/16**

(84) Benannte Vertragsstaaten: **AT CH DE FR GB IT LI LU NL SE**

(56) Entgegenhaltungen:
**CH-A- 644 833**
**DE-A- 1 957 680**
**DE-A- 2 817 576**
**DE-A- 3 212 106**
**US-A- 2 667 517**
**US-A- 3 285 967**

**PATENT ABSTRACTS OF JAPAN, unexamined applications, C Feld, Band 7, Nr. 28, 4. Februar 1983 THE PATENT GOVERNMENT Seite 137 C 149**
**PATENT ABSTRACTS OF JAPAN, unexamined applications, C Feld, Band 7, Nr. 114, 18. Mai 1983 THE PATENT OFFICE JAPANESE GOVERNMENT Seite 17 C 166**

(73) Patentinhaber: **Degussa Aktiengesellschaft, Weissfrauenstrasse 9, D-6000 Frankfurt am Main 1(DE)**

(72) Erfinder: **Arntz, Dietrich, Dr. Dipl.-Chem., Lorsbachstrasse 32, D-6370 Oberursel(DE)**
Erfinder: **Baacke, Michael, Dr. Dipl.-Chem., Grünaustrasse 19, D-6450 Hanau 9(DE)**
Erfinder: **Kleinschmit, Peter, Dr. Dipl.-Chem., Wildaustrasse 19, D-6450 Hanau 9(DE)**
Erfinder: **Prescher, Günter, Dr. Dipl.-Chem., Liesingstrasse 2, D-6450 Hanau 9(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung ungesättigter Ether durch katalytische Dealkoxylierung von geminalen Di-alkoxyverbindungen zur Herstellung von Vinylethern, die Verwendung von Zeolithen für diesen Zweck und Verfahren zu deren Herstellung.

Die katalytische Umsetzung von Acetalen zu ungesättigten Äthern ist seit langem bekannt. Nach Reppe et al., Ann. 601, 81–84 (1956) (nach C.A. 51: 9578 b–f) setzt man z.B. einen Silber/Asbest-Katalysator ein. Auch gemäß DE-OS 1 957 680 (C.A. 75: 76155 y) werden edelmetallhaltige Katalysatoren für diesen Zweck verwendet. Diese zeigen zwar eine befriedigende Selektivität, gleichzeitig aber auch schon bei Einsatz einfach gebauter Acetale geringe Umsätze. Dazu kommt als weiterer Nachteil ihr hoher Preis.

Die US-PS 3 285 967 betrifft die katalytische Dealkoxylierung an Trilithiumorthophosphat-haltigen Katalysatoren. Es werden aber keine Aussagen über deren Standzeiten gemacht.

Die Untersuchung einer Anzahl weiterer verschiedener Katalysatoren auf ihre Eignung bei der Herstellung von Vinylethern findet sich bei I.A. Bogod et al. (Khim Prom. 1972, 48(9), 657–60 übersetzt in Soviet Chem. Ind. no 9, 1972, 547–549).

Diese Veröffentlichung enthält neben Angaben über die Selektivität und Ausbeute als einzige auch Daten, die bei der Untersuchung der für die Praxis sehr wesentlichen Standzeiten der Katalysatoren ermittelt wurden.

Am stabilsten zeigte sich danach ein Kalium-ausgetauschter Zeolith des Typs A, der auch nach 1000 Stunden noch eine Selektivität von 99% besaß, das allerdings bei einem nur 60%igen Umsatz.

Aufgabe der Erfindung ist ein Verfahren zur Herstellung von Vinylethern aus Acetalen an einem Katalysator, der bei langen Standzeiten gleichzeitig hohen Umsatz und große Selektivität garantiert.

Gegenstand der Erfindung ist ein Verfahren zur Herstellung ungesättigter Ether der allgemeinen Formel

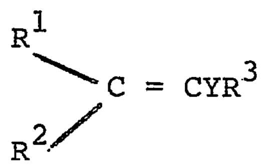

(I)

durch katalytische Dealkoxylierung von geminalen Dialkoxyverbindungen der Formel (II)

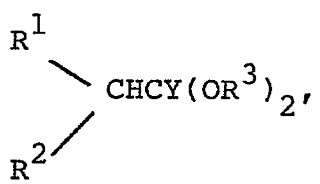

(II)

wobei jeweils bedeuten:
$R^1$, $R^2$: $C_1$–$C_3$-Alkyl, Aryl, H,
$R^3$: Methyl, Ethyl
Y: H, Methyl, bei erhöhter Temperatur in der Gasphase an einem Na-ausgetauschten Zeolith als Katalysator, das dadurch gekennzeichnet ist, daß man Zeolithe des Typs Mordenit oder ZSM5 mit einem $Na_2O/Al_2O_3$-Mol-Verhältnis von 1 : 1,05 (± 0,25) einsetzt.

Als Produkte erhält man Vinylmethyl- und Vinylethylether und den Ausgangsverbindungen entsprechend substituierte Vinylmethyl- und Vinylethylether. Die Reaktion läuft nach dem folgenden Schema ab:

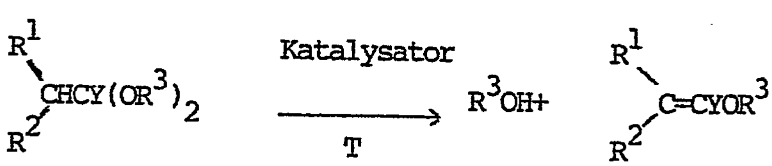


in dem $R^1$, $R^2$, $R^3$ und Y die oben angegebenen Bedeutungen haben.

Bevorzugt eingesetzt werden die Dimethyl- und Diäthylacetale des Acetaldehyds, Propionaldehyds und Butyraldehyds, Isobutyraldehyds und Dimethylketal. Die Umsetzung gelingt aber auch mit Dimethylphenylacetaldehydacetal.

Die Reaktion findet bei Temperaturen zwischen 200 und 450°C, bevorzugt 260 und 360°C, insbesondere zwischen 280 und 340°C statt. Der Reaktionsdruck liegt so niedrig, daß die Acetale bzw. Ketale unter den Reaktionsbedingungen nicht auf dem Katalysator kondensieren, im allgemeinen zwischen 0 und 2 atü, vorzugsweise arbeitet man ohne Überdruck.

Als Katalysatoren setzt man Zeolithe des Typs Mordenit oder ZSM5 ein, die ein $Na_2O/Al_2O_3$-Molverhältnis von 1:1,05 (±0,25) bevorzugt 1:1,05 (±0,15), insbesondere 1:1,05 (±0,05) aufweisen.

Die Typisierung der eingesetzten Zeolithe wird gemäß Breck "Zeolithe Molecular Sieves", John Wiley & Sons, 1974, S. 373 und S. 231 anhand der Röntgenbeugungsdiagramme vorgenommen.

Der Beispielteil enthält zwei Verfahren gemäß dem Stand der Technik (DE-OS 3 212 106, DE-OS 2 817 576), nach denen man Zeolithe des Typs ZSM5 und Mordenit herstellen kann. Sie können aber auch anders hergestellt werden.

Die so erhaltenen Zeolithe werden nach an sich bekannten Verfahren in die H-Form überführt und anschließend mit Natronlauge so behandelt, daß im Endprodukt ein $Na_2O/Al_2O_3$-Molverhältnis von 1:1,05 (±0,25), bevorzugt 1:1,05 (±0,15), insbesondere 1:1,05 (±0,05) resultiert.

Zu diesem Zweck stellt man eine 5 bis 40 gew.-%ige Suspension des in die H-Form überführten Zeoliths, bezogen auf die Gesamtmenge der Suspension, in einer wäßrigen Natriumhydroxidlösung her, rührt 0,1 bis 20 h bei 20 bis 100°C, bevorzugt 0,5–5 h bei 50–80°C, und dampft anschließend bis zur Trockne ein. Dabei ist die Konzentration des Natriumhydroxids so bemessen, daß das gewünschte Na/Al-Molverhältnis im Endprodukt erreicht wird (Imprägnierung). Die Reaktionszeit sinkt mit steigender Temperatur, bevorzugt setzt man den Zeolith bei 70–80°C innerhalb von 2–3 h um.

Ein anderer Weg zur Herstellung des erfindungsgemäß eingesetzten Katalysators besteht darin, daß man eine 5 bis 40 gew.-%ige Suspension eines in die H-Form überführten Mordenits bzw. ZSM5-Zeoliths in Wasser herstellt und anschließend unter Kontrolle des pH-Wertes der Suspension eine wäßrige 2 bis 50 gew.-%ige, bevorzugt 5 bis 10 gew.-%ige, Natronlauge bei 20 bis 100°C, bevorzugt 50 bis 80°C, innerhalb von 0,1 bis 10 h, bevorzugt 0,5 bis 5 h, unter Rühren zusetzt (Ionenaustausch). Solange sich der pH-Wert nicht ändert, wird Natronlauge zudosiert und dieser Vorgang erst beendet, wenn der pH-Wert sprunghaft ansteigt. Bei Vorhandensein geeigneter Apparaturen kann man natürlich auch unter Druck und bei höheren Temperaturen arbeiten.

Nach dem Abkühlen filtriert man den Feststoff ab und trocknet ihn bei 80 bis 200°C, bevorzugt 140°C. Der Wassergehalt beläuft sich dann auf 3 bis 20 Gew.-%. Obwohl auch die auf diesen Wegen in Pulverform anfallenden Katalysatoren nach der Temperung bei 250 bis 900°C (1 bis 20 h), bevorzugt 1 bis 5 h bei 600–800°C, prinzipiell die gewünschten Eigenschaften bei der Umsetzung der Acetale und Ketale zeigen, erweist sich erfahrungsgemäß z.B. in einem Festbettreaktor die Verwendung von zylindrischen oder kugelförmigen Pellets in den üblichen Abmessungen als zweckmäßig, während in einem Fließbettreaktor ein feinteiliges Katalysatorpulver einsetzbar ist.

Bei der Herstellung dieser Pellets ist es vorteilhaft, Verformungshilfsmittel wie z.B. Kieselsol in einer Menge von 1 bis 5 ml Kieselsol (40 Gew.-% $SiO_2$) pro 5 g Zeolith einzusetzen, der bevorzugt in ungetempertem Zustand vorliegt (~10 Gew.-% $H_2O$). Die Menge des Verformungsmittels kann innerhalb weiter Grenzen variiert werden. Dabei ist natürlich die untere Grenze zu berücksichtigen, bei der die Festigkeit des Formlings den mechanischen Beanspruchungen im Reaktor noch genügt und eine obere Grenze, bei der das katalytisch wirksame Material so stark verdünnt wird, daß der Umsatz spürbar absinkt. Im Anschluß an die an sich bekannte Verformung erfolgt die Trocknung bei 100–150°C und anschließend eine Temperung der Pellets, bevorzugt für 0,5 bis 20 h auf 300 bis 800°C.

Die Reaktivierung eines verbrauchten Katalysators auf die Anfangsselektivität kann auf einfachem Wege in der Weise erfolgen, daß man Luft einer Temperatur von 400 bis 600°C, bevorzugt von 450 bis 530°C, durch den Reaktor leitet.

Es zeigt sich, daß man auch ohne Ausbeuteverluste Acetale enthaltende Mischungen einsetzen kann, die auch ohne die in der DE-OS 3 403 426 angegebene Extraktion aus der Reaktionslösung durch einfache Destillation gewonnen werden und z.B. im Falle von Dimethylacetal 25 bis 30 Gew.-% Methanol und 2 bis 5 Gew.-% Acetaldehyd enthalten; Mischungen, die in vielen Fällen zu einer erhöhten Bildung von Dimethylether als unerwünschtem Nebenprodukt führen.

Vor der Einleitung in den Reaktor kann man das Acetal bzw. Ketal auf die Reaktionstemperatur vorheizen. Es ist aber auch möglich, dem Katalysator die notwendige Wärme durch Vorheizen zuzuführen. Man belastet den Katalysator bevorzugt mit 0,05 bis 3 kg, insbesondere 0,3 bis 1,5 kg Acetal pro kg Katalysator und Stunde.

Die aus dem Reaktor abströmenden Reaktionsgase werden anschließend direkt in einer nachgeschalteten Kolonne rektifiziert. Nicht umgesetzte Acetale bzw. Ketale und bei der Reaktion gebildeter sowie von Anfang an vorhandener Alkohol bleiben im Sumpf zurück.

Mit dem erfindungsgemäßen Verfahren erzielt man lange Standzeiten bei gleichzeitig konstant hoher Selektivität und hohem Umsatz, wenn die Na-ausgetauschten Mordenite und ZSM5-Zeolithe eingesetzt werden. Nach dem Stand der Technik (Bogod et al.) waren im Gegensatz dazu gerade bei Verwendung eines Na-ausgetauschten Y-Zeolithen eine nur sehr geringe Standzeit bzw. Selektivität zu registrieren gewesen.

Beispiele

Beispiel 1

Herstellung des Mordenit-Typs

a) Zu einer Suspension von 33,1 kg gefällter Kieselsäure (89% $SiO_2$) in 220 l $H_2O$ wird eine Lösung von 2,07 kg NaOH und 4,26 kg Natriumaluminat (34,35% $Na_2O$, 46,54% $Al_2O_3$) in 20 l $H_2O$ gegeben. Die Mischung wird in einem 300-l-Autoklaven 77 h bei 180°C gerührt, das kristalline Produkt abfiltriert und mit 300 l Wasser gewaschen. Nach dem Röntgenbeugungsdiagramm handelt es sich um gut kristallinen Zeolith vom Mordenit-Typ

Analyse 5,29% $Na_2O$
9,57% $Al_2O_3$
73,56% $SiO_2$
10,25% Gewichtsverlust (1000°C)

b) Zur Überführung in die H-Form werden 2 kg Na-Mordenit aus Beispiel 1 in 20 l 2N $H_2SO_4$ suspendiert, 2 h bei 80°C gerührt und abfiltriert. Die Behandlung wird zweimal wiederholt

Analyse 0,30% $Na_2O$
6,57% $Al_2O_3$
74,64% $SiO_2$
16,53% Gewichtsverlust (1000°C)

Beispiel 2

Herstellung des ZSM5-Typs

a) Eine Lösung von 0,56 kg NaOH und 0,65 kg Natriumaluminat (39,35% $Na_2O$, 46,54% $Al_2O_3$) in 40 l $H_2O$ wird zu einer Suspension von 25 kg gefällter Kieselsäure (89% $SiO_2$) in einer Lösung von 10,76 kg 1,6-Diaminohexan in 220 l $H_2O$ gegeben. Die Mischung wird 70 h bei 180°C gerührt, abfiltriert, mit 300 l $H_2O$ gewaschen und 24 h bei 120°C getrocknet. Das Produkt besteht aus gut kristallisiertem Zeolith vom Strukturtyp ZSM5.

Analyse 0,56% $Na_2O$
1,65% $Al_2O_3$
94,26% $SiO_2$
1,80% Gewichtsverlust (1000°C)

b) Zur Überführung in die H-Form werden 1 kg ZSM5 nach Beispiel 2 in 10 l 1N $H_2SO_4$ 2 h bei 80°C gerührt und anschließend abfiltriert. Die Behandlung wird noch einmal wiederholt.

Analyse 0,02% $Na_2O$
1,60% $Al_2O_3$
93,65% $SiO_2$
2,05% Gewichtsverlust (1000°C)

Beispiel 3

Die in die H-Form überführten Zeolithe werden durch Imprägnierung (Katalysatoren A–D, G, H) bzw. durch Ionenaustausch (Katalysator J) in die erfindungsgemäß einsetzbare Na-Form überführt.

Imprägnierung (Katalysatoren A–D, G, H)

Zeolith, der in der H-Form vorliegt, wird in einer wäßrigen NaOH-Lösung suspendiert, 2 h bei 80°C gerührt und anschließend bis zur Trockne eingedampft.

Ionenaustausch (Katalysator J)

Zeolith, der in der H-Form vorliegt, wird in Wasser suspendiert. Dabei stellt sich ein pH-Wert von ~4 ein. Innerhalb von 2 h bei 80°C gibt man 1N Natronlauge solange zu, bis der pH-Wert stark ansteigt. Nach dem Abkühlen filtriert man ab und trocknet bei 140°C.

Verformung

Die erhaltenen Zeolithpulver A–D, G–J werden mit Kieselsol (LUDOX® HS 40 (40% $SiO_2$)) zu einer verformbaren Masse verarbeitet (0,475 ml Kieselsol/g Zeolith) und mit Hilfe eines Alexanderwerk-Granulators zu zylindrischen Pellets (Durchmesser 3 cm, Länge 2–4 mm) verformt, anschließend 16 h bei 125°C getrocknet und 4 h bei 450°C getempert.

In Tabelle 1 sind die $Na_2O/Al_2O_3$ Verhältnisse der nach den obenangegebenen Vorschriften hergestellten Katalysatoren A–D; G–J und die zugehörigen Mengenverhältnisse der Reaktanten enthalten:

Tabelle 1

| Katalysator | g Zeolith | g NaOH | ml $H_2O$ | Molverhältnis $Na_2O/Al_2O_3$ im Endprodukt |
|---|---|---|---|---|
| A | 500 g Mordenit | 3,0 | 1000 | 0,75 |
| B | 600 g Mordenit | 8,3 | 1000 | 0,95 |
| C | 600 g Mordenit | 10,7 | 1000 | 1,05 |
| D | 600 g Mordenit | 11,9 | 1000 | 1,10 |
| G | 500 g ZSM5 | 5,65 | 1000 | 0,95 |
| H | 500 g ZSM5 | 6,20 | 1000 | 1,05 |
| J | 1200 g Mordenit | 86,35 | 2500 | 1,03 |

Die mit den in Tabelle 1 aufgeführten Katalysatoren erzielten Ergebnisse sind den nachfolgenden Beispielen zu entnehmen.

Beispiel 4

Umsetzung von Acetaldehyddimethylacetal (DMA)

1640 g des nach Beispiel 3 hergestellten Katalysators J wurden in einen durch elektrische Beheizung auf 310°C Reaktionstemperatur gehaltenen 3 m langen Reaktor mit einem Durchmesser von 29 mm eingefüllt, anschließend 800 g Acetaldehyddimethylacetal (DMA) pro Stunde über den Katalysator geleitet und die Reaktionsgase direkt in einer nachgeschalteten Kolonne rektifiziert. Nach 50 Stunden Betriebszeit erhielt man bei einem Umsatz von 93% am Kopf der Kolonne mit einer Selektivität von 99,5% Vinylmethylether in einer Reinheit von 99,7%. Nach 1000 Stunden Betriebszeit waren Umsatz und Selektivität immer noch konstant. In beiden Fällen wurden 6,9% DMA, bezogen auf Einsatz DMA, und 92,6% Methanol im Sumpf der Kolonne zurückgewonnen.

Beispiel 5

Umsetzung eines Gemisches aus DMA, Methanol und Acetaldehyd

960 g des nach Beispiel 3 hergestellten Katalysators J wurden in einem auf 300°C temperierten Reaktor von 2 m Länge und einem Durchmesser von 29 mm eingefüllt, anschließend 690 g Gemisch mit einem Gehalt von 24,5% Gew.-% Methanol, 2,1 Gew.-% Acetaldehyd und 73,4 Gew.-% DMA pro Stunde durch den Reaktor geleitet und das Reaktionsgas in einer Kolonne direkt destillativ aufgetrennt. Am Kopf der Kolonne wurde mit einer Selektivität von 99,1%, bezogen auf die 90,6% des umgesetzten DMA, Vinylmethylether isoliert. Neben Vinylmethylether enthielt das Destillat 2,8 Gew.-% Acetaldehyd. Nicht umgesetztes DMA und gebildetes sowie eingespeistes Methanol verblieben vollständig im Sumpf.

Beispiel 6

40 g des nach Beispiel 3 hergestellten Katalysators J wurde in einem mit einem Salzbad auf 300°C temperierten Reaktor mit 12 mm Durchmesser gefüllt. Bei einer Einspeisung von 60 ml DMA pro Stunde ergab sich ein Umsatz von 88% mit einer VME-Selektivität von 99%.

Beispiel 7 bis 13

Entsprechend Beispiel 6 wurden folgende Katalysatoren eingesetzt:

Tabelle 2

| Beispiel Nr. | Katalysator | | Reaktions-temperatur (°C) | Umsatz (%) | VME-Selektivität (%) |
|---|---|---|---|---|---|
| | $Na_2O$ | $Al_2O_3$ | | | |
| 7 | D | 1,10 | 340 | 91 | 98,3 |
| 8 | C | 1,05 | 300 | 82 | 96,8 |
| 9 | H | 1,05 | 300 | 89 | 95,4 |
| 10 | B | 0,95 | 280 | 87 | 96,2 |
| 11 | A | 0,75 | 280 | 93 | 91,3 |
| 12 | G | 0,95 | 300 | 93 | 92,4 |
| 13 | J | 1,03 | 300 | 88 | 99,0 |

Beispiel 14 bis 18

Entsprechend Beispiel 6 wurden anstelle von DMA weitere Acetale zu Vinylethern umgesetzt. Es wurde der Katalysator C gemäß Beispiel 3 eingesetzt.
Tabelle 3 zeigt die erhaltenen Ergebnisse:

Tabelle 3

Synthese verschiedener Vinylether

Beispiel 14–18
Reaktorwandtemperatur: 300°C
Katalysatorbelastung: 1,5 kg Acetal (Ketal)/kg Katalysator/h

| Acetal (Ketal) | Ether | Umsatz (%) | Ether-Selektivität (%) |
|---|---|---|---|
| 14 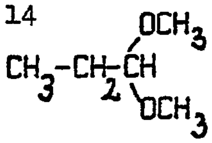 | $CH_3-CH=CH-OCH_3$ | 97 | 98,0 |
| 15 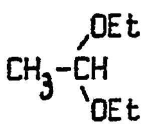 | $CH_2=CH-OET$ | 98 | 80 |
| 16 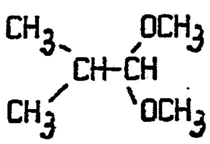 | 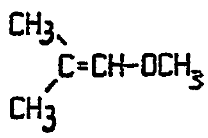 | 93 | 77 |
| 17 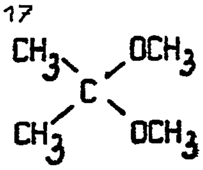 | 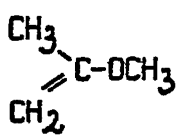 | 93 | 90 |
| 18 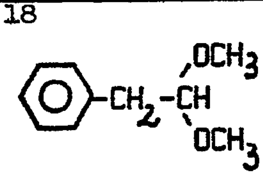 | 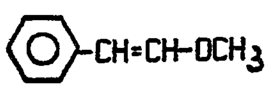 | 40 | 25 |

**Patentansprüche**

1. Verfahren zur Herstellung ungesättigter Ether der allgemeinen Formel

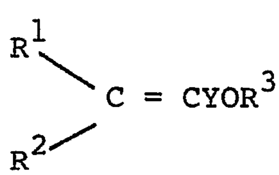 (I)

durch katalytische Dealkoxylierung von geminalen Dialkoxyverbindungen der Formel (II)

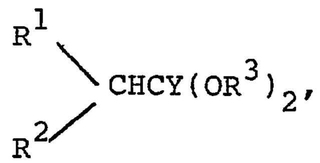

(II)

wobei jeweils bedeuten:
$R^1$, $R^2$: $C_1$–$C_3$-Alkyl, Aryl, H,
$R^3$: Methyl, Ethyl
Y: H, Methyl, bei erhöhter Temperatur in der Gasphase an einem Na-ausgetauschten Zeolith als Katalysator, dadurch gekennzeichnet, daß man Zeolithe des Typs Mordenit oder ZSM5 mit einem $Na_2O/Al_2O_3$-Mol-Verhältnis von 1:1,05 (± 0,25) einsetzt.

2. Verwendung von Zeolithen des Typs Mordenit oder ZSM5 mit einem $Na_2O/Al_2O_3$-Mol-Verhältnis von 1:1,05 (±0,25) als Katalysatoren bei der Herstellung von ungesättigten Ethern der allgemeinen Formel (I) durch Dealkoxylierung von geminalen Di-alkoxyverbindungen gemäß der allgemeinen Formel (II).

3. Verfahren zur Herstellung eines Katalysators gemäß den Ansprüchen 1 und 2, dadurch gekennzeichnet, daß man einen H-ausgetauschten Zeolith des Typs Mordenit oder ZSM5 in Wasser suspendiert, anschließend unter Kontrolle des pH-Werts eine wäßrige Natriumhydroxidlösung unter Rühren zusetzt, bis der pH-Wert sprunghaft ansteigt, nach dem Abkühlen den Feststoff abfiltriert, ihn trocknet und bei erhöhter Temperatur tempert.

## Claims

1. A process for the production of unsaturated ethers corresponding to the following general formula

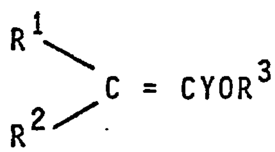

(I)

by catalytic dealkoxylation of geminal dialkoxy compounds corresponding to formula (II)

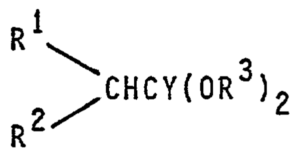

(II)

$R^1$ and $R^2$ representing $C_1$–$C_3$ alkyl, aryl, H,
$R^3$ representing methyl, ethyl,
Y representing H, methyl,
at elevated temperature in the gas phase on an Na-exchanged zeolite as catalyst, characterized in that zeolites of the mordenite or ZSM5 type with an $Na_2O:Al_2O_3$ molar ratio of 1:1.05 (±0.25) are used.

2. The use of zeolites of the mordenite or ZSM5 type with an $Na_2O/Al_2O_3$ molar ratio of 1:1.05 (±0.25) as catalysts in the production of unsaturated ethers corresponding to general formula (I) by dealkoxylation of geminal dialkoxy compounds corresponding to general formula (II).

3. A process for the production of a catalyst as claimed in claims 1 and 2, characterized in that an H-exchanged zeolite of the mordenite or ZSM5 type is suspended in water, an aqueous sodium hydroxide solution is then added with stirring and monitoring of the pH value until there is a sudden increase in pH, the solid is filtered off after cooling, dried and then conditioned at elevated temperature.

## Revendications

1. Procédé de production d'éthers non saturés de formule générale:

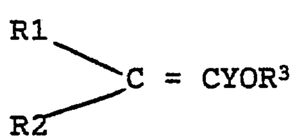

(I)

par désalcoxylation catalytique de composés dialcoxylés géminaux de formule (II):

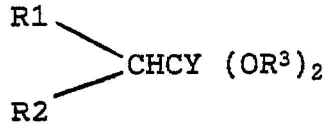

(II)

dans laquelle respectivement signifient:

$R_1$, $R_2$: alcoyle en $C_1$–$C_3$, aryle, hydrogène

$R_3$: méthyle ou éthyle

Y: hydrogène, méthyle

à température élevée en phase gazeuse sur une zéolite ayant échangé son sodium, comme catalyseur, caractérisé en ce que l'on met en œuvre une zéolite du typ mordenite ou ZSM5 avec un rapport $Na_2O/Al_2O_3$ de 1:1,05 (±0,25).

2. Utilisation de zéolites du type mordenite ou ZSM5 ayant un rapport molaire $Na_2O/Al_2O_3$ de 1:1,05 (±0,25) comme catalyseur pour la production d'éthers non saturés de formule générale I, par désalcoxylation de composés dialcoxylés germinaux selon la formule générale (II).

3. Procédé de préparation d'un catalyseur selon les revendications 1 et 2, caractérisé en ce que l'on met en suspension une zéolite du type mordenite ou ZSM5 convertie en forme $H^+$, dans de l'eau, ensuite on ajoute sous contrôle de la valeur du pH une solution aqueuse d'hydroxyde de sodium tout en agitant jusqu'à ce que la valeur du pH montre d'un seul coup, séparé par filtration après refroidissement, la substance solide, la sèche et la calcine à température élevée.